# EUROPEAN PATENT APPLICATION

(11) **EP 2 441 443 A1**
(43) Date of publication of application: **18.04.2012**
(21) Application number: 10186852.9
(22) Date of filing: 07.10.2010
(51) Int. Cl.: A61K 9/19, A61K 9/50, A61K 38/16, A61K 9/00, A61P 31/04, A61P 31/12, A61P 3/10, A61P 5/50, A61P 1/14, A61P 3/00, A61P 37/04

(54) **Lactoferrin based complex coacervates and their uses**

(71) Applicant: Nestec S.A., 1800 Vevey (CH)
(72) Inventor: Bovetto, Lionel, 74500 Larringes (FR); Breuille, Denis, 1010 Lausanne (FR); Donato-Capel, Laurence, 1033 Cheseaux-sur-Lausanne (CH); Pouteau, Etienne, 1005 Lausanne (CH); Schmitt, Christophe, 1077 Servion (CH)
(74) Representative: Marquardt, Ulf

(57) **Abstract**

Lactoferrin-based complex coacervate inducing delayed protein digestion and modified peptide profile for improving metabolic responses, gut inflammatory response, satiety and food intake in humans, in association with an increase in GLP-1 and PYY gut hormones.

## Description

The present invention generally relates to the field of complex coacervates, in particular to the field of complex coacervates that can be used in the food industry. An embodiment of the present invention relates to a composition containing a complex coacervate comprising lactoferrin and at least one other protein with an isoelectric point lower than pH 7.0. It was found that such complexes may be used to delay protein digestion, satiety and food intake in humans.

Proteins may yield bioactive peptides during intestinal digestion. This digestion usually takes place in the proximal part of the intestine.

However, in direct contact to L-cells of the distal part of the intestine, the ileum and the colon, these bioactive peptides can stimulate GLP-1 (glucagon-like peptide 1) and PYY (peptide YY) release.

GLP-1 has been described to improve metabolic control and PYY was shown to improve satiety and food intake.

Delaying the digestion of proteins from the proximal part to more distal parts of the intestine, e.g., the ileum, could subsequently produce bioactive peptides directly in the ileum. This would then help to induce GLP-1 and PYY secretion into the whole body circulation more effectively, resulting finally in an improved metabolic control and reduced food intake.

A composition would be desirable that allows an improved metabolic control and a reduced food intake. This composition should contain proteins which yield bioactive peptides during intestinal digestion, and the bioactive peptides should be generated in a distal part of the intestine.

EP2074891 A1 is describing a satiety-inducing food composition, which is fluid at neutral pH and shows and increase in viscosity at acid pH, wherein the composition comprises at least a negatively loaded carbohydrate and at least a protein with an iso-electric point of about pH 3.5-5.5.

WO07029484A is providing a complex between lactoferrin and a nonpeptidic highly hydrophilic molecule, which has a lowered antigenicity and a pepsin-resistance imparted thereto.

WO0601659A is providing a complex between lactoferrin and a high-molecular weight electrolyte that is improving the stability of lactoferrin in presence of water and gastric juice.

Ye A. and Singh H. [2006, Journal of Colloid and Interface Science, 295, 249-254] are describing the formation of complexes between lactoferrin and β-lactoglobulin at the interface of soya emulsions prepared at pH 3.0 or 7.0.

Lesmes U. et al. [2010, Journal of Agricultural and Food Chemistry, 58, 7962] are describing corn oil emulsions stabilised by complexes between lactoferrin and sodium caseinate. These emulsions are exhibiting an improved physical stability in the pH range 3.0 to 7.0 but could still be rapidely disgested by lipase in a *in vitro* model.

Mann D.M. et al. [1994, The Journal of Biological Chemistry, 269, 23661] reported the formation of electrostatic complexes between human lactoferrin and sulphates glycosaminoglycans such as chondroitin sulphate and heparin. These complexes are likely to be involved in the *in vivo* inflammatory response induced by lactoferrin.

No protein-protein complexes have been described that can be used to delay protein digestion in the intestine. Moreover, it would be desirable if such complexes were liquid in nature since this would facilitate their incorporation into food products.

Hence, it was the object of the present invention to provide the art with a protein based composition, that is easy to prepare and to incorporate into food products and that exhibits a delayed digestion, so that it allows an improved metabolic control and a reduced food intake.

The present inventors were surprised to see that complex coacervates between lactoferrin and oppositely charged proteins can be used to achieve the objective of the present invention.

The formation of complex coacervates between lactoferrin and oppositely charged proteins allows modulating the digestion kinetics and the residual native protein content of the oppositely charged protein after digestion. Different hydrolysate compositions may be produced after protein digestion using the teachings of the present invention. These phenomena were found to allow improving metabolic responses and gut inflammatory response, increase satiety and decrease food intake in humans, in association with an increase of GLP-1 and PYY secretion.

Complex coacervates are well defined in the art.

Cooper et al. [Current Opinion in Colloid and Interface Science, (2005), 10, 52-78] are defining complex coacervation by the separation of a macromolecular solution, composed of at least two macromolecules (typically oppositely charged polyelectrolytes), into two immiscible liquid phases. In this case, the coacervate is defined as either the macroscopic phase concentrated in macromolecules obtained after phase separation or the liquid droplets concentrated in macromolecules obtained after mixing the two dispersions containing oppositely charged macromolecules, i.e. proteins in this specific case. Thermodynamically, complex coacervates are formed by the aggregation of macromolecular complexes that are formed between two oppositely charges macromolecules (protein or polysaccharide) in order to reduce the free energy of the mixture as pointed out by Schmitt et al. [Handbook of Hydrocolloids, Second Edition. Woodhead Publishing, 2009, pp. 420-476]. Generally, macromolecular complexes and subsequent formation of coacervates are mediated by electrostatic interactions, and formation of coacervates occurs when aggregates of macromolecular complexes are reaching the electrostatic limit of colloidal stability, i.e. the surface ζ-potential is between -15 and +15 mV.

The present inventors have found that complex coacervates that can be used to achieve the object of the present invention are formed between lactoferrin and oppositely charged proteins, e.g., beta-lactoglobulin, alpha-lactalbumin, bovine serum albumin, whey protein isolate, lupin protein isolate, egg white protein isolate, soy protein isolate, pea protein, and/or potato protein isolate. Conditions for formation of these coacervates may include: pH 4.0 to 8.0, total protein concentration: 0.5 to 5 wt%, weight mixing ratio 5:1 and 1:5, ionic strength: 0-0.1 M and temperature: 4 to 50°C. The resulting complex coacervates are characterized by their liquid nature, an average diameter of more than 500 nm and a ζ-potential between + 15 and -15 mV.

Consequently, the present invention relates to a composition containing a complex coacervate comprising lactoferrin and at least one other protein with an isoelectric point lower than pH 7.0.

In principle, any other protein with an isoelectric point lower than pH 7.0 will produce suitable coacervates. For food applications, such a protein should be edible. Compounds are considered edible if they are generally approved for human or animal consumption.

Any suitable dietary protein may be used, for example animal proteins (such as milk proteins, meat proteins and egg proteins); vegetable proteins (such as soy protein, wheat protein, rice protein, and pea protein); or combinations thereof. Milk proteins such as whey proteins; and soy proteins are particularly preferred.

The choice of the other protein in the complex coacervate may further be used to influence stability, taste, texture and/or further functionalities of the complex coacervate.

The at least one other protein in the complex coacervate may selected from the group consisting of beta-lactoglobulin, alpha-lactalbumin, bovine serum albumin, whey protein isolate, lupin protein isolate, egg white protein isolate, soy protein isolate, potato protein isolate, pea protein isolate and combinations thereof.

For example, the lactoferrin and the at least one other protein with an isoelectric point lower than 7.0 may both be obtained from natural sources, such as milk, for example.

For an improved texture and an easier handling the complex coacervate may be present in a liquid form.

The size of the complex coacervate will have an effect on the delay of protein digestion. Larger complex coacervates will generally produce the bioactive peptides at a later stage than smaller complex coacervates.

Hence, the dimensions of the complex coacervates may be used to influence digestion kinetics.

For example, the complex coacervate may have a diameter of at least 500 nm, at least 750 nm or at least 1000 nm in the shortest dimension.

The complex coacervate may have a ζ-potential of between + 15 and -15 mV, for example between + 10 and -10 mV, or between +7 and -7 mV.

The complex coacervates of the present invention may be formed using any method known in the art.

Typically, the complex coacervates may be formed at a pH in the range of 4.0 to 8.0, e.g., 5.0 to 7.0, or 5.5 to 6.5.

Complex formation may take place at an ionic strength in the range of 0 to 0.1 M, for example 0.02 to 0.08 M, e.g., 0.04 to 0.06 M.

The temperature may be in the range of 1 to 50°C, e.g., 10 to 40°C or 15 to 25°C.

The total protein concentration may be in the range of 0.5 to 5 wt%, e.g., 1 to 4 wt%, or 2 to 3 wt%.

Lactoferrin and the at least one other protein may be present in a weight ratio in the range of 5:1 to 1:5, e.g., 3:1 to 1:3, or 2:1 to 1:2.

Hence, in one embodiment of the present invention the complex coacervate is formed at a pH in the range of 5.0 to 7.0, at an ionic strength in the range of 0 to 0.1 M, at a temperature in the range of 1 to 50°C, and with a total protein concentration in the range of 0.5 to 5 wt%, while lactoferrin and the at least one other protein are present in a weight ratio in the range of 5:1 to 1:5.

In another embodiment of the present invention the complex coacervate is formed at a pH in the range of 4.0 to 8.0, at an ionic strength in the range of 0.02 to 0.08 M, at a temperature in the range of 10 to 40°C, and with a total protein concentration in the range of 1 to 4 wt%, while lactoferrin and the at least one other protein are present in a weight ratio in the range of 3:1 to 1:3.

In a further embodiment of the present invention the complex coacervate is formed at a pH in the range of 5.5 to 6.5, at an ionic strength in the range of 0.04 to 0.06 M, at a temperature in the range of 15 to 25°C, and with a total protein concentration in the range of 2 to 3 wt%, while lactoferrin and the at least one other protein are present in a weight ratio in the range of 2:1 to 1:2.

The composition of the complex coacervate may be adjusted to modulate the biological response.

Modulating the kinetics of digestion will modulate amount of peptide released into the lumen of the gut impacting the strength and duration of the biological effect.

Using varying proteins in the complex will lead to different peptidic profiles, e.g., the formation of different biologically active peptides in different amounts.

Modulation the residual native protein amount will also have an impact on the biological response. In particular large amounts of residual native protein will produce an improved feeling of satiety.

Consequently, the composition of the present invention may be provided in different conditions which effect, e.g., the kinetics of protein digestion.

For example, the composition may have a pH in the range of 4.0 to 8.0, e.g., 5.0 to 7.0, or 5.5 to 6.5.

The composition may have an ionic strength in the range of 0 to 0.1 M, for example 0.02 to 0.08 M, e.g., 0.04 to 0.06 M.

The composition may be provided at a temperature in the range of 1 to 50°C, e.g., 10 to 40°C or 15 to 25°C.

The total protein concentration in the composition may be in the range of 0.5 to 5 wt%, e.g., 1 to 4 wt%, or 2 to 3 wt%.

Lactoferrin and the at least one other protein may be present in the composition in a weight ratio in the range of 5:1 to 1:5, e.g., 3:1 to 1:3, or 2:1 to 1:2.

Hence, in one embodiment of the present invention the composition has a pH in the range of 5.0 to 7.0, an ionic strength in the range of 0 to 0.1 M, a temperature in the range of 4 to 50°C, and a total protein concentration in the range of 0.5 to 5 wt%, while lactoferrin and the at least one other protein are present in a weight ratio in the range of 5:1 to 1:5.

In another embodiment of the present invention the composition has a pH in the range of 4.0 to 8.0, an ionic strength in the range of 0.02 to 0.08 M, a temperature in the range of 10 to 40°C, and a total protein concentration in the range of 1 to 4 wt%, while lactoferrin and the at least one other protein are present in a weight ratio in the range of 3:1 to 1:3.

In a further embodiment of the present invention the composition has a pH in the range of 5.5 to 6.5, an ionic strength in the range of 0.04 to 0.06 M, a temperature in the range of 15 to 25°C, and a total protein concentration in the range of 2 to 3 wt%, while lactoferrin and the at least one other protein are present in a weight ratio in the range of 2:1 to 1:2.

The composition of the present invention may be provided in a dried form, for example as a powder.

Such dried form will allow an easy disability and will increase storage times and - of course - will decrease the weight of the composition.

Hence, the composition may be subjected to a drying step, such as spray-drying or a freeze-drying.

After consumption of the composition of the present invention bioactive peptides will be generated in the distal part of the intestine. There, e.g., GLP-1 (glucagon-like petide 1) and PYY (peptide YY) release is stimulated leading to an improved metabolic control and a feeling of satiety and a reduced food intake.

Consequently, the composition of the present invention may be for use in the treatment or prevention of metabolic disorders, in particular overweightness or obesity.

It may also be used to support weight management.

The composition of the present invention may also be used in increasing satiation and/or prolonging satiety.

The present invention also extends to the use of the composition of the present invention for the preparation of a product to treat or prevent metabolic disorders, in particular overweightness or obesity.

"Overweight" is defined for an adult human as having a BMI between 25 and 30.

"Body mass index" or "BMI" means the ratio of weight in kg divided by the height in metres, squared.

"Obesity" is a condition in which the natural energy reserve, stored in the fatty tissue of animals, in particular humans and other mammals, is increased to a point where it is associated with certain health conditions or increased mortality. "Obese" is defined for an adult human as having a BMI greater than 30.

The composition of the present invention may alternatively or additionally be for use in the treatment or prevention of gut inflammatory disorders, such as inflammatory bowel disease, for example.

The composition of the present invention may alternatively or additionally be for use in boosting the immune system, in particular in the treatment or prevention of bacterial infections.

The complex coacervates of the present invention allow increasing the bioavailability of lactoferrin. Hence, the invention extends to a composition comprising the complex coacervates of the present invention for use in increasing the bioavailability of lactoferrin.

The composition may be any kind of composition. Preferably, the composition is a composition that is intended to be administered orally or enterally to animals, e.g., pet animals, or humans.

The composition may be selected from the group consisting of food compositions, pet food compositions, drinks, nutritional formulas or nutraceuticals.

The composition may be to be administered to people that wish to manage their body weight, in particular to avoid weight gain.

The composition may be to be administered to people who wish to lose weight, e.g., overweight or obese people.

One advantage of the present invention is that the complex coacervates are entirely natural products without additives and are well suitable for all age groups.

Hence, the composition of the present invention may be to be administered to children, e.g., infants."Infants" are children younger than 12 months.

Those skilled in the art will understand that they can freely combine all features of the present invention described herein, without departing from the scope of the invention as disclosed. In particular, features described for the uses of the present invention may be applied to the composition of the present invention and vice versa.

Further advantages and features of the present invention are apparent from the following Examples and Figures.

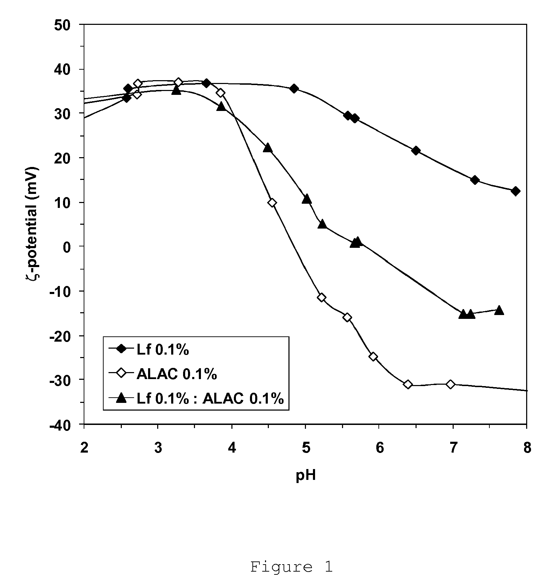

Figure 1 shows a titration curve showing the variation of the ζ-potential as a function of pH for lactoferrine (LF), a-lactalbumin (ALAC) and a LF/ALAC mixture at 0.1 wt% (total protein concentration) and 25°C. It is shown than the zeta-potential of LF is positive over the whole pH range tested. It does decrease when the pH is increased close to the isoelectric point (IEP) of the protein (about pH 9.0). To the contrary, ALAC is positively charged for a pH below its IEP, i.e. 5.0. The ζ-potential then turns into negative for higher pH values. The ζ-potential of the mixture at a 1:1 weight mixing ratio shows electroneutrality at pH 5.7. This point is corresponding to the optimum for complex coacervation to occur, with the maximum yield of coacervate formed. Nevertheless, coacervates are formed in the pH range of 4.5 to 6.0 because the overall ζ-potential is lower than 20 mV in absolute value. This does not lead to colloidal stability of soluble complexes that are forming coacervates.

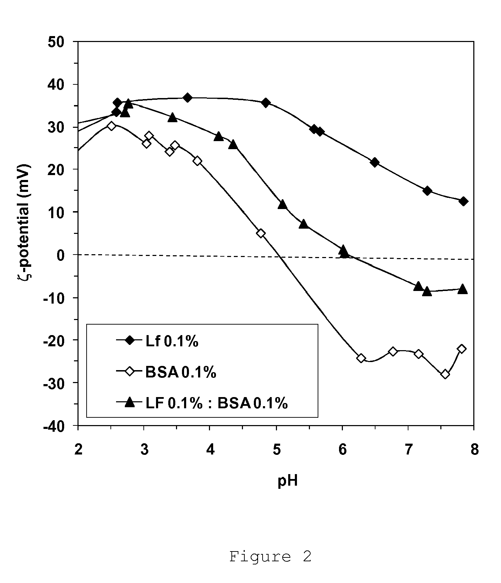

Figure 2 shows a titration curve showing the variation of the ζ-potential as a function of pH for LF, Bovine Serum Albumin (BSA) and a LF/BSA mixture at 0.1 wt% (total protein concentration) and 25°C. BSA is positively charged for a pH below its IEP, i.e. 5.1. The ζ-potential then turns into negative for higher pH values. The ζ-potential of the mixture at a 1:1 weight mixing ratio shows electroneutrality at pH 6.1. This point is corresponding to the optimum for complex coacervation to occur, with the maximum yield of coacervate formed. Nevertheless, coacervates are formed for pH values higher than 5.0 because the overall ζ-potential is lower than 20 mV in absolute value. This does not lead to colloidal stability of soluble complexes that are forming coacervates.

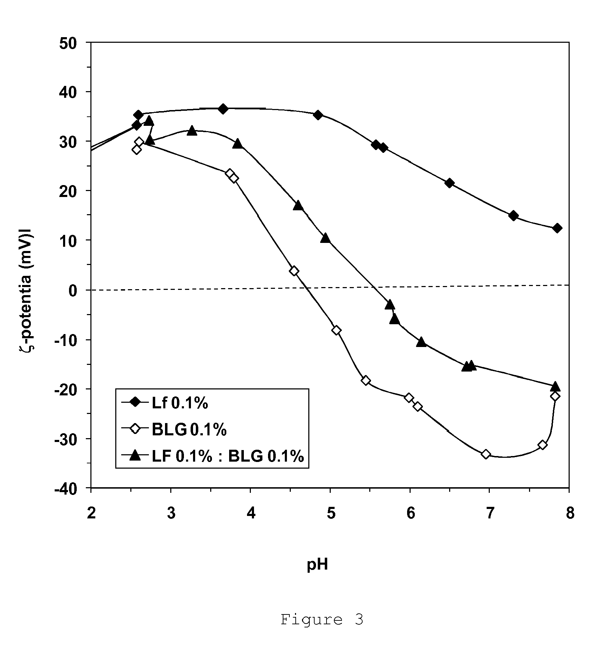

Figure 3 shows a titration curve showing the variation of the ζ-potential as a function of pH for LF,B-lactoglobulin (BLG) and a LF/BLG mixture at 0.1 wt% (total protein concentration) and 25°C. BLG is positively charged for pH below its IEP, i.e. 4.5. The ζ-potential then turns into negative for higher pH values. The ζ-potential of the mixture at a 1:1 weight mixing ratio shows electroneutrality at pH 5.6. This point is corresponding to the optimum for complex coacervation to occur, with the maximum yield of coacervate formed. Nevertheless, coacervates are formed on the pH range from 4.8 to 6.8 because the overall ζ-potential is lower than 20 mV in absolute value. This does not lead to colloidal stability of soluble complexes that are forming coacervates.

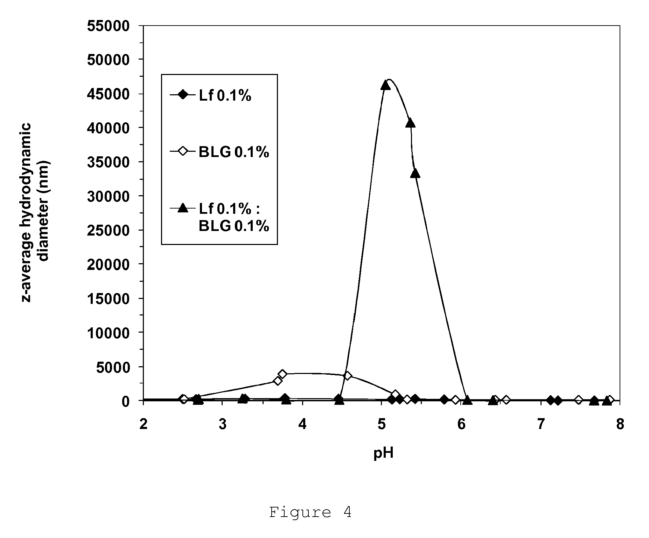

Figure 4 shows the variation of the z-average hydrodynamic diameter of particles as a function of pH for Lf, BLG and a LF/BLG mixture at 0.1 wt% (total protein concentration) and 25°C. It can be seen that the size of the particles is strongly increasing when the LF/BLG mixture is entering the pH range of 4.5 to 6.1. In this case, microscopic complex coacervates are formed due to the low z-potential of the complexes that do aggregate. On the contrary, LF/BLG soluble complexes are formed outside this pH range, leading to submicroscopic particles. Particle size increase in the BLG sample is due to self-aggregation close its IEP of 4.5.

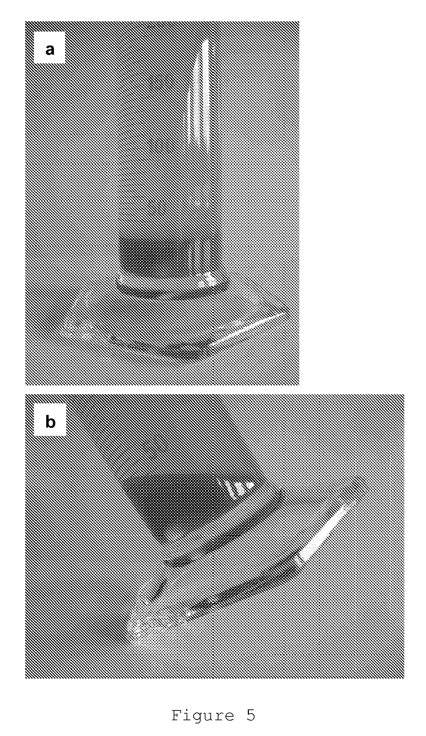

Figure 5 shows the macroscopic appearance of the coacervate phase and the corresponding equilibrium phase obtained 24 h after mixing 200 mL of BLG at 2 wt% and 200 mL of LF at 2 wt% at pH 5.9 and 25°C. (A) Coacervate volume. (B) Evidence of liquid nature of coacervate phase after 45° tube tilting. It can be seen that about 25 mL of coacervate are obtained upon mixing a 200 mL of a 2 wt% LF dispersion with 200 mL of 2 wt% BLG dispersion at pH 5.9. A yield of coacervation of 70% was estimated by measurement of protein content in the soluble fraction (by HPLC analysis). Initial total protein content was equal to 8g. Kjeldhal titration showed that 2.4g were recovered in the soluble phase corresponding to 375 mL. Protein concentration in the coacervate phase can be estimated close to 22.4% (estimated 5.6g in 25 mL), which result in a concentration factor of 11. The liquid nature of the coacervate can be clearly seen from the tilting of the glass tube.

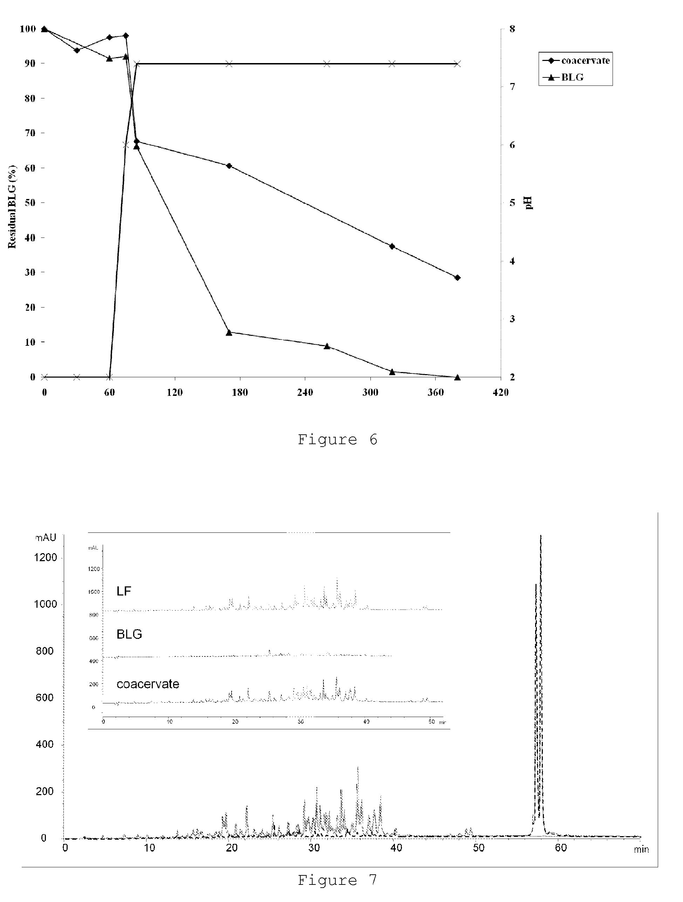

Figure 6 shows the residual BLG estimate from HPLC integration as function of time (min) and pH for selected systems if *in vitro* digested. Different kinetic profiles of residual BLG as function of time of an *in vitro* digestion are observed for mixtures of LF/BLG forming coacervates. At pH 2, BLG is weakly hydrolyzed in constrast to LF which is completely hydrolyzed. At pH 7.4, the residual BLG amount is higher for the coacervate than for BLG alone. The present result illustrates a way to decrease rate of BLG digestion. Increasing the amount of residual non digested BLG at the end of digestion could impact biological response at the distal part of the intestine and play a role in the regulation of the satiety response.

Figure 7 shows an HPLC-C18 chromatogram of selected systems at kinetic points of in vitro digestion T60pH2. Full line: Coacervate LF6%/BLG6%, dotted line: BLG 5%, pointed line LF 5%. Window enlarges peptide profile with some differences highlighted in grey rectangle. A grey rectangle indicates an example of differences that could be observed in the chromatogram. These results illustrate the quantitative and qualitative differences in peptides that are generated depending on the initial system composition (coacervate, BLG o r LF). Different peptides profiles induce different biological responses.

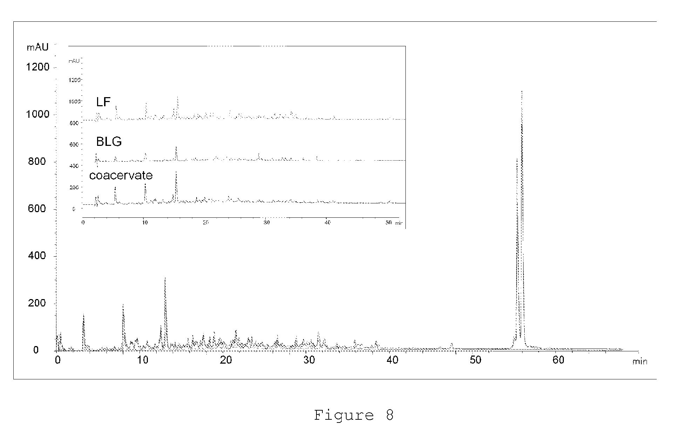

Figure 8 shows an HPLC-C18 chromatogram of selected systems at kinetic points of in vitro digestion T5pH7.4. Full line: Coacervate LF6%/BLG6%, dotted line: BLG 5%, pointed line LF 5%. Window enlarges peptide profile with some differences highlighted in grey rectangle. A grey rectangle indicates an example of differences that could be observed in the chromatogram. These results illustrate the quantitative and qualitative differences in peptides that are generated depending on the initial system composition (coacervate, BLG or LF). Different peptides profiles induce different biological responses.

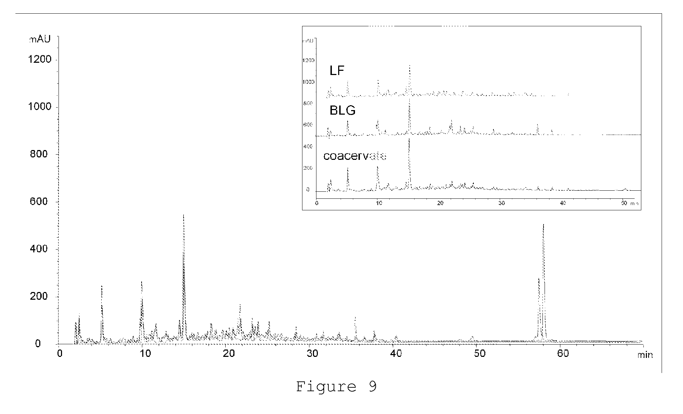

Figure 9 shows an HPLC-C18 chromatogram of selected systems at kinetic points of in vitro digestion T300pH7.4. Full line: Coacervate LF6%/BLG6%, dotted line: BLG 5%, pointed line LF 5%. Window enlarges peptide profile with some differences highlighted in grey rectangle. A grey rectangle indicates an example of differences that could be observed in the chromatogram. These results illustrate the quantitative and qualitative differences in peptides that are generated depending on the initial system composition (coacervate, BLG o r LF). Different peptides profiles induce different biological responses.

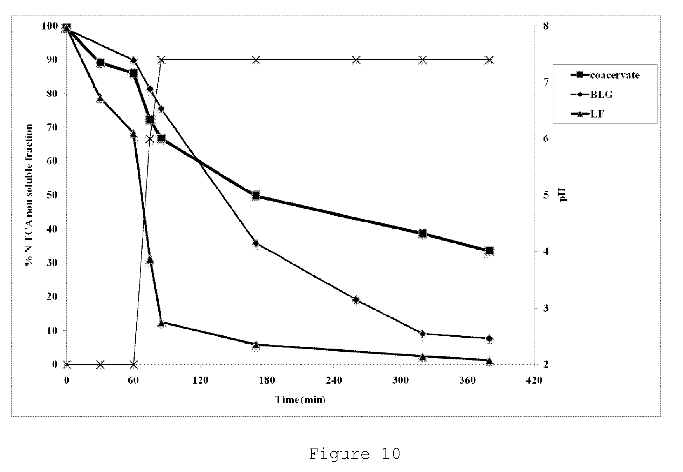

Figure 10 shows the N-TCA (Nitrogen non soluble in trichloro acetic acid) non soluble fraction as a function of time and pH of in vitro digestion for the coacervate LF/BLG, BLG 5% and LF 5%. Different kinetics profiles of N-TCA non soluble fraction as a function of steps of in vitro digestion are observed for mixtures of LF/BLG forming coacervates. In the form of coacervate, larger N-TCA non soluble fractions are measured. Considering that the N-TCA soluble fraction could be associated to the fraction of peptides that is transferred to the blood, the results suggest that a lower peptide fraction amount is transferred when a LF/BLG mixture is administrated in the form of coacervate compared to BLG alone. Larger residual fraction remaining could offer the possibility to interact with gut receptors at the distal part of the intestine to stimulate biological response.

### Materials

β-Lactoglobulin (BLG) (lot JE 001-8-415) was purchased from Davisco Foods International Inc. (Le Sueur, MN, USA). The powder contained 89.6 wt% protein (Kjeldhal analysis: N x 6.38).

α-Lactalbumin (ALAC) (JE 031-8-410) was purchased from Davisco Foods International Inc. (Le Sueur, MN, USA). The powder contained 88.8wt% protein (Kjeldhal analysis: N x 6.38).

Bovine serum albumin (BSA) (lot 035K0573) was purchased from Sigma (St Louis, MO, USA). The powder contained 98 wt% protein (Kjeldhal analysis: N x 6.38).

Lactoferrin (LF) (lot 104-44-427) was purchased from DMV (The Netherlands). The powder contained 93.1 wt% protein (Kjeldhal analysis: N x 6.38).

### Determination of the ζ-potential in BLG/Lf mixtures

The ζ-potential of the single protein, or LF/protein mixtures was determined by light scattering upon application of an alternating electrical field into the measuring cell at a protein concentration of 0.1 wt% after filtration on 0.22 µm filters. The dispersions at pH 8.0 were placed in a electrophoretic mobility cell and analyzed at a scattering angle of 173° using the Nanosizer ZS (Malvern Instruments, UK). The effective electrical field, E, applied to the measurement cell was between 50 and 150 V depending on the conductivity of the samples. The overall electrophoretic mobility of the particles was determined and the corresponding ζ-potential (V) was then calculated using the Smoluchowski equation. The ζ-potential was determined on the pH-range from 8.0 to 2.0 upon titration of the protein dispersion with 0.01 and 0.1 M hydrochloric acid.

### Determination of the particle size in BLG/Lf mixtures

Particle size was determined by dynamic light scattering (DLS) using a Nanosizer ZS (Malvern Instruments, UK). The apparatus is equipped with a He-Ne laser emitting at 633 nm and with a 4.0 mW power source. The instrument uses a backscattering configuration where detection is done at a scattering angle of 173° using an avalanche photodiode. The protein dispersions were diluted to 0.1 wt% in Millipore water and filtered on 0.22 µm filters. Samples at pH 8.0 were then poured in the electrophoretic mobility cell connected to a MPT-2 titrator unit. Measurements were performed at 25°C. Depending on the sample turbidity the pathlength of the light was set automatically by the apparatus. The autocorrelation function G2(t) was calculated from the fluctuation of the scattered intensity with time. From the polynomial fit of the logarithm of the correlation function using the "cumulants" method, the z-average hydrodynamic diameter of the particles was calculated assuming that the diffusing particles were monodisperse spheres. The particle size was determined on the pH-range from 8.0 to 2.0 upon titration of the protein dispersion with 0.01 and 0.1 M hydrochloric acid.

### Macroscopic observation of BLG/Lf coacervate phase

A coacervate was prepared by mixing 200 mL of a BLG dispersion at 2 wt% (protein basis) at pH 5.9 with 200 mL of a LF dispersion at 2 wt% at pH 5.9 in a glass cylinder. The mixture was then left at rest for 24 h at 25°C and a macroscopic phase separation was observed. The liquid nature of the coacervate phase was probed by tilting the glass cylinder. Pictures were taken using a digital camera.

### In-vitro digestion

Protein solutions were prepared by dispersing 5 wt% BLG or LF powder (based on protein content) in Millipore water (resistivity: 18.2 MΩ.cm), and stirring for 1 h at 20°C. The solution was then gently stirred at 4°C for 12h to allow complete hydration.

Coacervates of LF/BLG were prepared by mixing in 4 centrifugal tubes 200g of LF 4 wt% solution adjusted at pH5.9 with NaOH 1M and 200g of BLG 4 wt% solution adjusted at pH 5.9 with HCl 1M. After mixing, the solution were stored 3 days at 4°C and then centrifuged at 4°C at 15888g (Sorval Evolution, Rotor SLA 3000). Supernatant was removed and replaced with 40g of mineral ballast and water to reach final weight of 120g. The protein concentration of coacervate was estimated by Kjeldhal analysis (N*6.38, see details below) and was equal to 12.3% by weight with a ratio 1:1 of LF/BLG as estimated from HPLC analysis.

### Digestion method

Due to the possible heterogeneity of the sample, in vitro digestion of protein solution is performed in different glass vials corresponding to the different points of kinetics namely *IN-PH2, T30-pH2, T60-pH2, T15-pH6, T5-pH7.4, T90-pH7.4, T180-pH7.4, T240-pH7.4, T300-pH7.4.* Each point is done in duplicate allowing two different treatments of the samples.

Pepsin (Pepsin porcine gastric mucosa, Sigma P7000, batch 118K0023) is dispersed in mineral ballast with a final concentration of 20mg/mL. Pancreatin (Pancreatin porcine pancreas, Sigma P7545, batch 0088K0763) and bile (Bile extract porcine, Sigma B8631, batch 058K0066) are dispersed separately in MilliQ water with final concentration of 20mg/mL and 40mg/mL respectively. 20 min before use, pancreatin and bile solution are centrifuged at 2800g for 10 min (Allegra, Beckman Coulter) and supernatant is used for in-vitro digestion.

For pH adjustment, NaOH, HCl (Merck, Zurich, Switzerland), and NaH₂PO₄ 2H₂O (Sigma-Aldrich, Steinheim, Germany) 1M pH 8.0 buffer are used.

A mineral ballast was used to for in vitro digestion composed of 1.35 mM, CaCl₂ , 2H₂O (Fluka, Steinheim, Germany), 7.14 mM NaHCO₃ (Fluka, Steinheim, Germany) adjust at pH2.0.

In a first stage, the specific volume V1 of HCl, V3, V5 NaOH and V2, V4 NaH₂PO₄ buffer (with V2+V4 = 2 mL) to reach pH of stomach (pH2.0) and intestine (pH7.4) and pH6.0 are determined in a separate experiment.

Then, 10 g of protein solution is weighted in each 40 mL glass vial (Supelco, Bellefonte, PA, USA). 5 mL of mineral ballast and V1 mL of HC1 1M is then added to reach final pH2.0. All vials are then left for 10 min at 37°C in water bath under gentle shaking to allow temperature to equilibrate. Sample *IN-pH2* is then collected. 0.5 mL of pepsin solution is added allowing a ratio E/S =1 % (based on 10 wt% protein). Samples *T30-pH2* and *T60-pH2* are collected after 30 and 60 min at pH2.0. Final pH after 60 min is checked to be below pH3.0. V2 NaH₂PO₄, buffer and V3 NaOH 1M are then added to reach pH6.0. Then 0.5 mL of pancreatin allowing a ratio E/S =1 % (based on 10 wt% protein) and 0.5 mL of bile allowing a ratio E/S =2 % (based on 10 wt% protein) are added. Sample *T15-pH6* is then collected after 15 min at pH6.0. V3 NaH₂PO₄ buffer and V4 NaOH 1M are then added to reach pH7.4. Sample T5-pH7.4 is collected after 5 min at pH7.4. Osmolality of the sample is then measured with a Micro-osmometer Model 3MO (Advanced Instrument Inc., Norwood, MS, USA) and adjusted to 300 mOsm by addition of V6 of water (with a maximum final volume of sample limited to 40 mL). Samples *T90-pH7.4, T180-pH7.4, T240-pH7.4, T300-pH7.4* are then collected after 90, 180, 240 and 300 min at *pH7.4* respectively.

### Analysis of digests

Each points of the kinetics correspond to two glass vials. The enzymatic reaction is stopped either by adding 10 mL Trifluoroacetic acid (TFA) 0.4 % (Pierce, Rockford, IL, USA) or 10 mL trichloroacid (TCA) 48 % (Merck, Zurich, Switzerland) when the sample is collected. Final sample volume is adjusted with MilliQ water to 40 mL to a final concentration of 0.1% TFA and 12 % TCA respectively.

### Determination of percentage of residual N-TCA non soluble fraction using the Kjeldhal method

Samples containing 12% TCA are stored for 12 h at 4°C then centrifuged at 2800 g (Allegra, Beckman Coulter) for 15 min. 10 to 15 mL of supernatant is collected and put in Kjeldhal tubes in duplicate. 10 mL of H₂SO₄ 95-97% (Merck, Zurich, Switzerland) are then added to Kjeldhal Copper Sulfate and potassium sulfate pills (Merck, Zurich, Switzerland).

Mineralization of the sample is then started by increasing temperature 110°C for 5min, 380°C 180min and then cooling to room temperature during 60min. After neutralization of mineralized samples (ammonium form of nitrogen) by addition of 32% NaOH, ammonia was released and titrated with HC1 0.1M. Same procedure is performed on 1 mL of initial protein solution allowing determining the percentage of residual N-TCA non soluble fraction.

### Fractionation of hydrolysates and identification of peptide

Samples containing 0.1% TFA are stored at -20°C until analysis. Samples are first thaw in at 4°C for 12h, then centrifuged at 5000g for 15 min (Sorval Evolution, Rotor SLA 3000). Samples are then diluted to 1-2.5 mg/mL with TFA0.1%. Samples were injected on a Jupiter C18 column (150*2 mm, 3 µm, Phenomenex, USA) equilibrated in 98% solvent A (0.1% TFA in H2O) and 2 % solvent B (0.1% TFA, 90% acetonitrile (Merck, Zurich, Switzerland), 9.9 % H₂O at 40 °C, connected to an injector system composed of an Agilent series 1100 apparatus equipped with an autosampler and UV detector.

The sample was eluted at a flow rate of 0.25 mL.min⁻¹ with a linear gradient up to 85 % solvent B. Detection was fixed at 214nm. Chromatograms were integrated using Agilent Chemstation data analysis software and protein concentrations were evaluated using calibration curves established with BLG initial solution.

## Claims

1. Composition containing a complex coacervate comprising lactoferrin and at least one other protein with an isoelectric point lower than pH 7.0.

2. Composition in accordance with claim 1, wherein at least one other protein in the complex coacervate is selected from the group consisting of beta-lactoglobulin, alpha-lactalbumin, bovine serum albumin, whey protein isolate, lupin protein isolate, egg white protein isolate, soy protein isolate, potato protein isolate, pea protein isolate and combinations thereof.

3. Composition in accordance with one of the preceding claims, wherein the complex coacervate has a diameter of at least 500 nm in the shortest dimension.

4. Composition in accordance with one of the preceding claims, wherein the complex coacervate has a ζ-potential of between + 15 and -15 mV.

5. Composition in accordance with one of the preceding claims, wherein the complex coacervate was formed at a pH in the range of 4.0 to 8.0, at an ionic strength in the range of 0 to 0.1 M, at a temperature in the range of 1 to 50°C, and with a total protein concentration in the range of 0.5 to 5 wt%, while lactoferrin and the at least one other protein are present in a weight ratio in the range of 5:1 to 1:5.

6. Composition in accordance with one of the preceding claims, wherein the composition has a pH in the range of 4.0 to 8.0, an ionic strength in the range of 0 to 0.1 M, a temperature in the range of 1 to 50°C, a total protein concentration in the range of 0.5 to 5 wt%, and/or lactoferrin and the at least one other protein present in a weight ratio in the range of 5:1 to 1:5.

7. Composition in accordance with one of the preceding claims that is further subjected to a drying step, such as spray-drying or a freeze-drying.

8. Composition in accordance with one of the preceding claims for use in the treatment or prevention of metabolic disorders, in particular overweightness, obesity, pre-diabetes or diabetes.

9. Composition in accordance with one of the preceding claims for use in improving insulin secretion/action and glucose control.

10. Composition in accordance with one of the preceding claims for use in increasing satiation, prolonging satiety and/or food intake.

11. Composition in accordance with one of the preceding claims for use in the treatment or prevention of gut inflammatory disorders.

12. Composition in accordance with one of the preceding claims for use in boosting the immune system, in particular in the treatment or prevention of bacterial infections.

13. Composition in accordance with one of the preceding claims for use in increasing the bioavailability of lactoferrin.

14. Composition in accordance with one of the preceding claims, wherein the composition is selected from the group consisting of food compositions, pet food compositions, drinks, nutritional formulas or nutraceuticals; and/or wherein the composition is to be administered to infants and/or children.

15. Composition in accordance with one of the preceding claims, wherein the lactoferrin and the at least one other protein with an isoelectric point lower than 7.0 are obtained from natural sources, such as milk.
